# EUROPEAN PATENT APPLICATION

(11) **EP 4 245 820 A1**
(43) Date of publication of application: **20.09.2023**
(21) Application number: 21891926.4
(22) Date of filing: 10.11.2021
(51) Int. Cl.: C09K 5/04

(54) **REFRIGERANT-CONTAINING COMPOSITION, USE THEREOF, REFRIGERATOR HAVING SAID COMPOSITION, AND OPERATING METHOD FOR REFRIGERATOR**

(30) Priority: 10.11.2020 JP 2020187532
(71) Applicant: DAIKIN INDUSTRIES, LTD., Osaka 530-0001 (JP)
(72) Inventor: KUSHIDA, Megumi, Osaka-shi, Osaka 530-0001 (JP); NAKAUE, Tsubasa, Osaka-shi, Osaka 530-0001 (JP)
(74) Representative: Hoffmann Eitle
(86) International application number: PCT/JP2021/041427
(87) International publication number: WO 2022/102681

(57) **Abstract**

An object of the present invention is to provide a novel mixed refrigerant. A composition comprising a refrigerant, wherein the refrigerant comprises HFC-143 and/or HCFO-1131, and further comprises a specific additional compound is provided to achieve the object.

## Description

### Technical Field

The present disclosure relates to a composition comprising a refrigerant, use of the composition, a refrigerating machine having the composition, and a method for operating the refrigerating machine.

### Background Art

1,1,2-Trifluoroethane (HFC-143) is useful in expansion agents for polyolefins and polyurethanes, aerosol propellants, refrigerants, heat transfer media, gaseous dielectrics, fire extinguishing agents, power cycle working fluids, polymerization media, particulate removal fluids, carrier fluids, buffing abrasive agents, and displacement drying agents (PTL 1). HFC-143 has a boiling point of about 4°C.

### Citation List

### Patent Literature

PTL 1: WO94/011460

### Summary of Invention

### Technical Problem

An object of the present disclosure is to provide a novel mixed refrigerant.

### Solution to Problem

### Item 1.

A composition comprising a refrigerant, the refrigerant comprising 1,1,2-trifluoroethane (HFC-143), wherein
(1) the refrigerant further comprises a first additional compound and/or
(2) the composition further comprises at least one compound selected from the group consisting of water, hydrogen chloride, and hydrogen fluoride, and
the first additional compound is at least one compound selected from the group consisting of methane, tetrafluoroethane (HFC-14), difluoromethane (HFC-32), fluoromethane (HFC-41), trans-1,2-difluoroethylene (HFO-1132(E)), cis-1,2-difluoroethylene (HFO-1132(Z)), chloromethane (CH3Cl), chlorotrifluoroethylene (HCFO-1113), 1,1-dichloro-1-fluoroethane (HCFC-141b), 1,1-dichloroethylene (HCO-1130a), trans-1,2-dichloroethylene (HCO-1130(E)), 1,2-dichloro-1-fluoroethylene (HCFO-1121), 1,1-dichloro-2-fluoroethylene (HCFO-1121a), 1,2-dichloro-1,1-difluoroethane (HCFC-132b), 1,1-dichloro-2,2-difluoroethane (HCFC-132a), 1,2-dichloro-1,2-difluoroethane (HCFC-132), cis-1,2-dichloroethylene (HCO-1130(Z)), 1,2,2-trichloro-1,1-difluoroethane (HCFC-122), 1,1,1-trichloroethane (HCC-140a), 1,1-dichloroethane (HCC-150a), 1,1,2-trichloro-1-fluoroethane (HCFC-131a), trichloroethylene (HCO-1120), 1,1,2-trichloro-2-fluoroethane (HCFC-131), 1,1,2-trichloroethane (HCC-140), 1,1,2,2-tetrachloro-1-fluoroethane (HCFC-121), tetrachloroethylene (HCO-1110), 1,1,1,2 tetrachloroethane (HCC-130a), 1,1,2,2-tetrachloroethane (HCC-130), 1,1,1,2,2-pentachloroethane (HCC-120), tetrachloromethane, 1,2,2-trichloro-1-fluoroethylene (HCFO-131a), 1-chloropropene, 2-chloropropene, 3-chloropropene, 1-chloro-2-fluoroethane (HCFC-151), 1-chloro-1-fluoroethane (HCFC-151a), 1,1,1-trichloro-2,2-difluoroethane (HCFC-122b), 1-chloroethylene (HCO-1140), and carbon dioxide.

### Item 2.

A composition according to Item 1, wherein the refrigerant comprises HFC-143 in an amount of 30 mass% or more based on the entire refrigerant.

### Item 3.

The composition according to Item 1, wherein the refrigerant further comprises a second additional compound, and the second additional compound is at least one compound selected from the group consisting of 1,1,2,2-tetrafluoromethane (HFC-134), 1-chloro-1,1-difluoroethane (HCFC-142b), and 1-chloro-2,2,2-trifluoroethane (HCFC-133a).

### Item 4.

A composition comprising a refrigerant,
wherein
the refrigerant comprises 1-chloro-2-fluoroethylene (HCFO-1131),
(1) the refrigerant further comprises an additional compound and/or
(2) the composition further comprises at least one compound selected from the group consisting of water, hydrogen chloride, and hydrogen fluoride,

the additional compound is at least one compound selected from the group consisting of methane, HFC-14, trifluoromethane (HFC-23), 1,1-difluoroethylene (HFO-1132a), HFO-1132(E), 1,1,1-trifluoroethane (HFC-143a), HFO-1132(Z), 1,1,1,2-tetrafluoroethane (HFC-134a), chloromethane (CH3Cl), 1,1,2,2-tetrafluoroethane (HFC-134), HCFC-1113, 1-chloro-1-fluoroethylene (HFO-1131a), 1,1-difluoroethane (HFC-152a), 1-chloro-1,1-fluoroethane (HCFC-142b), 1,1,2-trifluoroethane (HFC-143), 1-chloro-2,2,2-trifluoroethane (HFC-133a), 1-chloro-1,2,2-trifluoroethane (HFC-133), 1,1-chloro-2,2,2-trifluoroethane (HCFC-123), HCFC-141b, HCO-1130a, HCO-1130(E), 1-chloro-2,2-difluoroethane (HCFC-142), 1-chloro-1,2-difluoroethane (HCFC-142a), HCFO-1121, HCFO-1121a, HCFC-132b, HCFC-132a, HCFC-132, HCO-1130(Z), HCFC-122, HCC-140a, HCC-150a, HCFC-131a, HCO-1120, HCFC-131, HCC-140, HCFC-121, HCO-1110, HCC-130a, HCC-130, HCC-120 tetrachloromethane, HCFO-131a, 1-chloropropene, 2-chloropropene, 3-chloropropene, 1,2-dichloro-1-fluoroethylene (HCFO-141), HCFC-151, HCFC-151a, HCFC-122b, HCO-1140, 1-fluoroethylene (HFO-1141), and carbon dioxide,
and HFO-1131 is present in an amount of 30 mass% or more based on the entire refrigerant.

### Item 5.

The composition according to any one of claims 1 to 4, comprising at least one compound selected from the group consisting of water, hydrogen chloride, and hydrogen fluoride.

### Item 6.

Use of the composition according to any one of Items 1 to 5 as a refrigerant.

### Item 7.

A refrigerating machine comprising the composition according to any one of Items 1 to 5 as a working fluid.

### Item 8.

A method for operating a refrigerating machine, comprising the step of circulating the composition according to any one of Items 1 to 5 as a working fluid in a refrigerating machine.

### Advantageous Effects of Invention

The present disclosure provides a novel mixed refrigerant.

### Description of Embodiments

The present disclosure includes the following embodiments.

### Definition of Terms

In the present specification, the term "refrigerant" includes at least compounds that are specified in ISO 817 (International Organization for Standardization), and that are given a refrigerant number (ASHRAE number) representing the type of refrigerant with "R" at the beginning; and further includes refrigerants that have properties equivalent to those of such refrigerants, even though a refrigerant number is not yet given. Refrigerants are broadly divided into fluorocarbon compounds and non-fluorocarbon compounds in terms of the structure of the compounds. Fluorocarbon compounds include chlorofluorocarbons (CFC), hydrochlorofluorocarbons (HCFC), and hydrofluorocarbons (HFC). Non-fluorocarbon compounds include propane (R290), propylene (R1270), butane (R600), isobutane (R600a), carbon dioxide (R744), ammonia (R717), and the like.

In the present specification, the phrase "composition comprising a refrigerant" at least includes (1) a refrigerant itself (including a mixture of refrigerants), (2) a composition that further comprises other components and that can be mixed with at least a refrigeration oil to obtain a working fluid for a refrigerating machine, and (3) a working fluid for a refrigerating machine containing a refrigeration oil. In the present specification, of these three embodiments, the composition (2) is referred to as a "refrigerant composition" so as to distinguish it from a refrigerant itself (including a mixture of refrigerants). Further, the working fluid for a refrigerating machine (3) is referred to as a "refrigeration oil-containing working fluid" so as to distinguish it from the "refrigerant composition."

In the present specification, when the term "alternative" is used in a context in which the first refrigerant is replaced with the second refrigerant, the first type of "alternative" means that equipment designed for operation using the first refrigerant can be operated using the second refrigerant under optimum conditions, optionally with changes of only a few parts (at least one of the following: refrigeration oil, gasket, packing, expansion valve, dryer, and other parts) and equipment adjustment. In other words, this type of alternative means that the same equipment is operated with an alternative refrigerant. Embodiments of this type of "alternative" include "drop-in alternative," "nearly drop-in alternative," and "retrofit," in the order in which the extent of changes and adjustment necessary for replacing the first refrigerant with the second refrigerant is smaller.

The term "alternative" also includes a second type of "alternative," which means that equipment designed for operation using the second refrigerant is operated for the same use as the existing use with the first refrigerant by using the second refrigerant. This type of alternative means that the same use is achieved with an alternative refrigerant.

In the present specification, the term "refrigerating machine" refers to machines in general that draw heat from an object or space to make its temperature lower than the temperature of ambient air, and maintain a low temperature. In other words, refrigerating machines refer to conversion machines that gain energy from the outside to do work, and that perform energy conversion, in order to transfer heat from where the temperature is lower to where the temperature is higher.

### 1. Refrigerant 1

The refrigerant 1 of the present disclosure may comprise 1,1,2-trifluoroethane (HFC-143) and further comprise a first additional compound, wherein the first additional compound is at least one compound selected from the group consisting of methane, tetrafluoroethane (HFC-14), difluoromethane (HFC-32), fluoromethane (HFC-41), trans-1,2-difluoroethylene (HFO-1132(E)), cis-1,2-difluoroethylene (HFO-1132(Z)), chloromethane (CH3Cl), chlorotrifluoroethylene (HCFO-1113), 1,1-dichloro-1-fluoroethane (HCFC-141b), 1,1-dichloroethylene (HCO-1130a), trans-1,2-dichloroethylene (HCO-1130(E)), 1,2-dichloro-1-fluoroethylene (HCFO-1121), 1,1-dichloro-2-fluoroethylene (HCFO-1121a), 1,2-dichloro-1,1-difluoroethane (HCFC-132b), 1,1-dichloro-2,2-difluoroethane (HCFC-132a), 1,2-dichloro-1,2-difluoroethane (HCFC-132), cis-1,2-dichloroethylene (HCO-1130(Z)), 1,2,2-trichloro-1,1-difluoroethane (HCFC-122), 1,1,1-trichloroethane (HCC-140a), 1,1-dichloroethane (HCC-150a), 1,1,2-trichloro-1-fluoroethane (HCFC-131a), trichloroethylene (HCO-1120), 1,1,2-trichloro-2-fluoroethane (HCFC-131), 1,1,2-trichloroethane (HCC-140), 1,1,2,2-tetrachloro-1-fluoroethane (HCFC-121), tetrachloroethylene (HCO-1110), 1,1,1,2 tetrachloroethane (HCC-130a), 1,1,2,2-tetrachloroethane (HCC-130), 1,1,1,2,2-pentachloroethane (HCC-120), tetrachloromethane, 1,2,2-trichloro-1-fluoroethylene (HCFO-131a), 1-chloropropene, 2-chloropropene, 3-chloropropene, 1-chloro-2-fluoroethane (HCFC-151), 1-chloro-1-fluoroethane (HCFC-151a), 1,1,1-trichloro-2,2-difluoroethane (HCFC-122b), 1-chloroethylene (HCO-1140), and carbon dioxide.

HFC-143 is also effective as an intermediate to obtain HFO-1132.

The refrigerant 1 of the present disclosure preferably comprises HFC-143 in an amount of 30 mass% or more, more preferably 50 mass% or more, and even more preferably 90 mass% or more based on the entire refrigerant. The upper limit of the proportion of HFC-143 in the refrigerant 1 of the present disclosure based on the entire refrigerant is preferably 99.99 mass%, more preferably 99.75 mass%, and even more preferably 99.5 mass%.

The refrigerant 1 of the present disclosure comprises the first additional compound in a total amount of preferably 70 mass% or less, more preferably 50 mass% or less, and even more preferably 30 mass% or less based on the entire refrigerant.

The refrigerant 1 of the present disclosure may contain the following second additional compound. The second additional compound is at least one compound selected from the group consisting of 1,1,2,2-tetrafluoromethane (HFC-134), 1-chloro-1,1-difluoroethane (HCFC-142b), and 1-chloro-2,2,2-trifluoroethane (HCFC-133a) .

The refrigerant 1 of the present disclosure comprises the second additional compound in a total amount of 1 mass% or less, more preferably 0.5 mass% or less, and even more preferably 0.1 mass% or less based on the entire refrigerant. The composition containing the refrigerant of the present disclosure containing the second additional compound in this proportion has excellent stability and can be used as a starting material for production of 1,2-difluoroethylene (HFO-1132).

The refrigerant 1 of the present disclosure may further comprise, in addition to HFC-143, the first additional compound, and the second additional compound, other additional refrigerants. The refrigerant 1 of the present disclosure preferably comprises HFC-143, the first additional compound, and the second additional compound in a total amount of 99.5 mass% or more, more preferably 99.75 mass% or more, even more preferably 99.9 mass% or more, still even more preferably 99.99 mass% or more, and most preferably 99.999 mass% or more.

Such additional refrigerants are not limited, and can be selected from a wide range of refrigerants. The mixed refrigerant may comprise a single additional refrigerant, or two or more additional refrigerants.

The refrigerant 1 according to the present disclosure can be preferably used as a working fluid in a refrigerating machine.

### 2. Refrigerant 2

The refrigerant 2 of the present disclosure may comprise 1-chloro-2-fluoroethylene (HCFO-1131) and further an additional compound, wherein the additional compound is at least one compound selected from the group consisting of methane, HFC-14, trifluoromethane (HFC-23), 1,1-difluoroethylene (HFO-1132a), HFO-1132(E), 1,1,1-trifluoroethane (HFC-143a), HFO-1132(Z), 1,1,1,2-tetrafluoroethane (HFC-134a), chloromethane (CH₃Cl), 1,1,2,2-tetrafluoroethane (HFC-134), HCFC-1113, 1-chloro-1-fluoroethylene (HFO-1131a), 1,1-difluoroethane (HFC-152a), 1-chloro-1,1-fluoroethane (HCFC-142b), 1,1,2-trifluoroethane (HFC-143), 1-chloro-2,2,2-trifluoroethane (HFC-133a), 1-chloro-1,2,2-trifluoroethane (HFC-133), 1,1-chloro-2,2,2-trifluoroethane (HCFC-123), HCFC-141b, HCO-1130a, HCO-1130(E), 1-chloro-2,2-difluoroethane (HCFC-142), 1-chloro-1,2-difluoroethane (HCFC-142a), HCFO-1121, HCFO-1121a, HCFC-132b, HCFC-132a, HCFC-132, HCO-1130(Z), HCFC-122, HCC-140a, HCC-150a, HCFC-113, HCO-1120, HCFC-131, HCC-140, HCFC-121, HCO-1110, HCC-130a, HCC-130, HCC-120 tetrachloromethane, HCFO-131a, 1-chloropropene, 2-chloropropene, 3-chloropropene, 1,2-dichloro-1-fluoroethylene (HCFO-141), HCFC-151, HCFC-151a, HCFC-122b, HCO-1140, 1-fluoroethylene (HFO-1141), and carbon dioxide, and HFO-1131 is present in an amount of 30 mass% or more based on the entire refrigerant.

The refrigerant 2 of the present disclosure comprises HFO-1131 in an amount of 30 mass% or more, more preferably in an amount of 50 mass% or more, and even more preferably 90 mass% or more based on the entire refrigerant. The upper limit of the proportion of HFO-1131 based on the entire refrigerant in the refrigerant 2 of the present disclosure is preferably 99.99 mass%, more preferably 99.75 mass%, and even more preferably 99.5 mass%.

The refrigerant 2 of the present disclosure comprises the additional compound in a total amount of preferably 70 mass% or less, more preferably 50 mass% or less, and even more preferably 30 mass% or less based on the entire refrigerant.

The refrigerant 2 of the present disclosure may further comprise other additional refrigerants in addition to HFO-1131 and the additional compound. The refrigerant 2 of the present disclosure preferably comprises HFO-1131 and the additional compound in a total amount of 99.5 mass% or more, more preferably 99.75 mass% or more, even preferably 99.9 mass% or more, still even more preferably 99.99 mass% or more, and most preferably 99.999 mass% or more based on the entire refrigerant.

Such additional refrigerants are not limited, and can be selected from a wide range of refrigerants. The mixed refrigerant may comprise a single additional refrigerant, or two or more additional refrigerants.

The refrigerant 2 according to the present disclosure can be preferably used as a working fluid in a refrigerating machine.

### 3. Refrigerant Composition

The refrigerants 1 and 2 of the present disclosure may be sometimes referred to collectively as the "refrigerants of the present disclosure." The refrigerant composition according to the present disclosure comprises at least the refrigerant according to the present disclosure, and can be used for the same use as the refrigerant according to the present disclosure. Moreover, the refrigerant composition according to the present disclosure can be further mixed with at least a refrigeration oil to thereby obtain a working fluid for a refrigerating machine.

The refrigerant composition according to the present disclosure further comprises at least one other component in addition to the refrigerant according to the present disclosure. The refrigerant composition according to the present disclosure may comprise at least one of the following other components, if necessary. As described above, when the refrigerant composition according to the present disclosure is used as a working fluid in a refrigerating machine, it is generally used as a mixture with at least a refrigeration oil. Therefore, it is preferable that the refrigerant composition according to the present disclosure does not substantially comprise a refrigeration oil. Specifically, in the refrigerant composition according to the present disclosure, the content of the refrigeration oil based on the entire refrigerant composition is preferably 0 to 1 mass%, and more preferably 0 to 0.1 mass%.

### 3.1. Water

The refrigerant composition according to the present disclosure may contain a small amount of water. The water content of the refrigerant composition is preferably 0.1 mass% or less, and more preferably 0.05 mass% or less based on the entire refrigerant. A small amount of water contained in the refrigerant composition stabilizes double bonds in the molecules of unsaturated fluorocarbon compounds that can be present in the refrigerant, and makes it less likely that the unsaturated fluorocarbon compounds will be oxidized, thus increasing the stability of the refrigerant composition.

### 3.2. Hydrogen chloride and hydrogen fluoride

The refrigerant composition of the present disclosure may contain hydrogen chloride and/or hydrogen fluoride. The proportion of hydrogen chloride in the refrigerant composition is preferably 0.5 mass% or less, and 0.25 mass% or less based on the entire refrigerant. The proportion of hydrogen fluoride in the refrigerant composition is preferably 0.5 mass% or less, and more preferably 0.25 mass% or less based on the entire refrigerant.

### 3.3. Tracer

A tracer is added to the refrigerant composition according to the present disclosure at a detectable concentration such that when the refrigerant composition of the present disclosure has been diluted, contaminated, or undergone other changes, the tracer can trace the changes.

The refrigerant composition according to the present disclosure may comprise a single tracer, or two or more tracers.

The tracer is not limited, and can be suitably selected from commonly used tracers.

Examples of tracers include hydrofluorocarbons, hydrochlorofluorocarbons, chlorofluorocarbons, hydrochlorocarbons, fluorocarbons, deuterated hydrocarbons, deuterated hydrofluorocarbons, perfluorocarbons, fluoroethers, brominated compounds, iodinated compounds, alcohols, aldehydes, ketones, and nitrous oxide (N₂O). The tracer is particularly preferably a hydrofluorocarbon, a hydrochlorofluorocarbon, a chlorofluorocarbon, a hydrochlorocarbon, a fluorocarbon, or a fluoroether.

Specifically, the following compounds are preferable as the tracer.
FC-14 (tetrafluoromethane, CF₄)
HCC-40 (chloromethane, CH₃Cl)
HFC-23 (trifluoromethane, CHF₃)
HFC-41 (fluoromethane, CH₃Cl)
HFC-125 (pentafluoroethane, CF₃CHF₂)
HFC-134a (1,1,1,2-tetrafluoroethane, CF₃CH₂F)
HFC-134 (1,1,2,2-tetrafluoroethane, CHF₂CHF₂)
HFC-143a (1,1,1-trifluoroethane, CF₃CH₃)
HFC-143 (1,1,2-trifluoroethane, CHF₂CH₂F)
HFC-152a (1,1-difluoroethane, CHF₂CH₃)
HFC-152 (1,2-difluoroethane, CH₂FCH₂F)
HFC-161 (fluoroethane, CH₃CH₂F)
HFC-245fa (1,1,1,3,3-pentafluoropropane, CF₃CH₂CHF₂)
HFC-236fa (1,1,1,3,3,3-hexafluoropropane, CF₃CH₂CF₃)
HFC-236ea (1,1,1,2,3,3-hexafluoropropane, CF₃CHFCHF₂)
HFC-227ea (1,1,1,2,3,3,3-heptafluoropropane, CF₃CHFCF₃)
HCFC-22 (chlorodifluoromethane, CHClF₂)
HCFC-31 (chlorofluoromethane, CH₂ClF)
CFC-1113 (chlorotrifluoroethylene, CF₂=CClF)
HFE-125 (trifluoromethyl-difluoromethyl ether, CF₃OCHF₂)
HFE-134a (trifluoromethyl-fluoromethyl ether, CF₃OCH₂F)
HFE-143a (trifluoromethyl-methyl ether, CF₃OCH₃)
HFE-227ea (trifluoromethyl-tetrafluoroethyl ether, CF₃OCHFCF₃)
HFE-236fa (trifluoromethyl-trifluoroethyl ether, CF₃OCH₂CF₃)

The refrigerant composition of the present disclosure may comprise a tracer in a total concentration of about 10 parts per million by weight (ppm) to about 1000 ppm based on the entire refrigerant composition. The refrigerant composition of the present disclosure comprises a tracer in a total concentration of about 30 ppm to about 500 ppm, and more preferably about 50 ppm to about 300 ppm based on the entire refrigerant composition.

### 3.4 Ultraviolet Fluorescent Dye

The refrigerant composition according to the present disclosure may comprise a single ultraviolet fluorescent dye, or two or more ultraviolet fluorescent dyes.

The ultraviolet fluorescent dye is not limited, and can be suitably selected from commonly used ultraviolet fluorescent dyes.

Examples of ultraviolet fluorescent dyes include naphthalimide, coumarin, anthracene, phenanthrene, xanthene, thioxanthene, naphthoxanthene, fluorescein, and derivatives thereof. The ultraviolet fluorescent dye is particularly preferably either naphthalimide or coumarin, or both.

### 3.5. Stabilizer

The refrigerant composition according to the present disclosure may comprise a single stabilizer, or two or more stabilizers.

The stabilizer is not limited, and can be suitably selected from commonly used stabilizers.

Examples of stabilizers include nitro compounds, ethers, and amines.

Examples of nitro compounds include aliphatic nitro compounds, such as nitromethane and nitroethane; and aromatic nitro compounds, such as nitro benzene and nitro styrene.

Examples of ethers include 1,4-dioxane.

Examples of amines include 2,2,3,3,3-pentafluoropropylamine and diphenylamine.

Examples of stabilizers also include butylhydroxyxylene and benzotriazole.

The content of the stabilizer is not limited. Generally, the content of the stabilizer is preferably 0.01 to 5 mass%, and more preferably 0.05 to 2 mass%, based on the entire refrigerant.

### 3.6. Polymerization Inhibitor

The refrigerant composition according to the present disclosure may comprise a single polymerization inhibitor, or two or more polymerization inhibitors.

The polymerization inhibitor is not limited, and can be suitably selected from commonly used polymerization inhibitors.

Examples of polymerization inhibitors include 4-methoxy-1-naphthol, hydroquinone, hydroquinone methyl ether, dimethyl-t-butylphenol, 2,6-di-tert-butyl-p-cresol, and benzotriazole.

The content of the polymerization inhibitor is not limited. Generally, the content of the polymerization inhibitor is preferably 0.01 to 5 mass%, and more preferably 0.05 to 2 mass%, based on the entire refrigerant.

### 4. Refrigeration Oil-Containing Working Fluid

The refrigeration oil-containing working fluid according to the present disclosure comprises at least the refrigerant or refrigerant composition according to the present disclosure and a refrigeration oil, for use as a working fluid in a refrigerating machine. Specifically, the refrigeration oil-containing working fluid according to the present disclosure is obtained by mixing a refrigeration oil used in a compressor of a refrigerating machine with the refrigerant or the refrigerant composition. The refrigeration oil-containing working fluid generally comprises 10 to 50 mass% of refrigeration oil.

### 4.1. Refrigeration Oil

The composition according to the present disclosure may comprise a single refrigeration oil, or two or more refrigeration oils.

The refrigeration oil is not limited, and can be suitably selected from commonly used refrigeration oils. In this case, refrigeration oils that are superior in the action of increasing the miscibility with the mixture and the stability of the mixture, for example, are suitably selected as necessary.

The base oil of the refrigeration oil is preferably, for example, at least one member selected from the group consisting of polyalkylene glycols (PAG), polyol esters (POE), and polyvinyl ethers (PVE).

The refrigeration oil may further contain additives in addition to the base oil. The additive may be at least one member selected from the group consisting of antioxidants, extreme-pressure agents, acid scavengers, oxygen scavengers, copper deactivators, rust inhibitors, oil agents, and antifoaming agents.

A refrigeration oil with a kinematic viscosity of 5 to 400 cSt at 40°C is preferable from the standpoint of lubrication.

The refrigeration oil-containing working fluid according to the present disclosure may further optionally contain at least one additive. Examples of additives include compatibilizing agents described below.

### 4.2. Compatibilizing Agent

The refrigeration oil-containing working fluid according to the present disclosure may comprise a single compatibilizing agent, or two or more compatibilizing agents.

The compatibilizing agent is not limited, and can be suitably selected from commonly used compatibilizing agents.

Examples of compatibilizing agents include polyoxyalkylene glycol ethers, amides, nitriles, ketones, chlorocarbons, esters, lactones, aryl ethers, fluoroethers, and 1,1,1-trifluoroalkanes. The compatibilizing agent is particularly preferably a polyoxyalkylene glycol ether.

### 5. Method for Operating Refrigerating Machine

The method for operating a refrigerating machine according to the present disclosure is a method for operating a refrigerating machine using the refrigerant according to the present disclosure.

Specifically, the method for operating a refrigerating machine according to the present disclosure comprises the step of circulating the refrigerant according to the present disclosure in a refrigerating machine.

The embodiments are described above; however, it will be understood that various changes in forms and details can be made without departing from the spirit and scope of the claims.

### Examples

The present disclosure is described in more detail below with reference to Examples. However, the present disclosure is not limited to the Examples.

### Example 1

Table 1 shows the calculation results of the refrigeration cycle of compositions containing HFC-143 and various additional compounds. The calculation conditions are as follows:
evaporation temperature: 10°C, condensation temperature: 45°C,
degree of superheat: 5°C, degree of supercooling: 5°C,
compression efficiency: 70%

Peng-Robinson was used as a physical properties model.

**Table 1**

| Component | Composition (weight ratio) | COP | Refrigerating capacity |
|---|---|---|---|
| | | (HFC-245fa relative to conventional refrigerant) | |
| HFC-143/Methane | 99.9/0.1 | 1.007 | 1.581 |
| HFC-143/HFC-14 | 99.9/0.1 | 1.029 | 1.601 |
| HFC-143/HFC-32 | 99/1 | 1.026 | 1.634 |
| HFC-143/HFC-41 | 99/1 | 1.004 | 1.631 |
| HFC-143/HFO-1132(E) | 99/1 | 1.027 | 1.628 |
| HFC-143/HFO-1132(Z) | 99/1 | 1.029 | 1.617 |
| HFC-143/Chloromethane | 99/1 | 1.030 | 1.622 |
| HFC-143/HFC-134 | 99/1 | 1.030 | 1.610 |
| HFC-143/HCFO-1113 | 99/1 | 1.029 | 1.610 |
| HFC-143/HCFC-142b | 99/1 | 1.030 | 1.605 |
| HFC-143/HCFC-133a | 99/1 | 1.030 | 1.599 |
| HFC-143/HCFC-141b | 99/1 | 1.031 | 1.589 |
| HFC-143/HCO-1130a | 99/1 | 1.031 | 1.587 |
| HFC-143/HCO-1130(E) | 99/1 | 1.032 | 1.583 |
| HFC-143/HCFO-1121 | 99/1 | 1.031 | 1.588 |
| HFC-143/HCFO-1121a | 99/1 | 1.031 | 1.587 |
| HFC-143/HCFC-132b | 99/1 | 1.031 | 1.587 |
| HFC-143/HCFC-132a | 99/1 | 1.033 | 1.586 |
| HFC-143/HCFC-132 | 99/1 | 1.031 | 1.586 |
| HFC-143/HCO-1130(Z) | 99/1 | 1.032 | 1.581 |
| HFC-143/HCFC-122 | 99/1 | 1.032 | 1.588 |
| HFC-143/HCC-140a | 99/1 | 1.032 | 1.585 |
| HFC-143/HCC-150a | 99/1 | 1.032 | 1.582 |
| HFC-143/HCFC-131a | 99/1 | 1.033 | 1.586 |
| HFC-143/HCO-1120 | 99/1 | 1.033 | 1.585 |
| HFC-143/HCFC-131 | 99/1 | 1.037 | 1.579 |
| HFC-143/HCC-140 | 99/1 | 1.037 | 1.577 |
| HFC-143/HCFC-121 | 99/1 | 1.034 | 1.577 |
| HFC-143/HCO-1110 | 99/1 | 1.036 | 1.581 |
| HFC-143/HCFC-130a | 99/1 | 1.037 | 1.579 |
| HFC-143/HCC-130 | 99/1 | 1.040 | 1.575 |
| HFC-143/HCC-120 | 99/1 | 1.037 | 1.579 |
| HFC-143/ Tetrachloromethane | 99/1 | 1.032 | 1.587 |
| HFC-143/HCFC-131a | 99/1 | 1.033 | 1.586 |
| HFC-143/1-Chloropropene | 99/1 | 1.031 | 1.582 |
| HFC-143/HCFC-151 | 99/1 | 1.032 | 1.580 |
| HFC-143/HCFC-151a | 99/1 | 1.031 | 1.592 |
| HFC-143/HCFC-122b | 99/1 | 1.032 | 1.588 |
| HFC-143/HFO-1140 | 99/1 | 1.030 | 1.611 |
| HFC-143/Water | 99/1 | 1.038 | 1.550 |
| HFC-143/HCl | 99/1 | 0.998 | 1.619 |
| HFC-143/CO₂ | 99/1 | 0.990 | 1.596 |

### Example 2

Table 1 shows the calculation results of the refrigeration cycle of compositions containing HCFC-1131 and various additional compounds. The calculation conditions are as follows: evaporation temperature: 10°C, condensation temperature: 45°C, degree of superheat: 5°C, degree of supercooling: 5°C, compression efficiency: 70%

Peng-Robinson was used as a physical properties model.

**Table 2**

| Component | Composition (Mass ratio) | COP | Refrigerating capacity |
|---|---|---|---|
| | | (HFC-245fa relative to conventional refrigerant) | |
| HCFO-1131/Methane | 99.9/0.1 | 1.013 | 1.262 |
| HCFO-1131/HFC-14 | 99.8/0.2 | 1.039 | 1.285 |
| HCFO-1131/HFC-23 | 99/1 | 1.013 | 1.277 |
| HCFO-1131/HFO-1132a | 99/1 | 1.039 | 1.308 |
| HCFO-1131/HFO-1132(E) | 99/1 | 1.039 | 1.308 |
| HCFO-1131/HFC-143a | 99/1 | 1.038 | 1.301 |
| HCFO-1131/HFO-1132(Z) | 99/1 | 1.041 | 1.303 |
| HCFO-1131/HFC-134a | 99/1 | 1.041 | 1.297 |
| HCFO-1131/ Chloromethane | 99/1 | 1.041 | 1.306 |
| HCFO-1131/HFC-134 | 99/1 | 1.040 | 1.316 |
| HCFO-1131/HCFO-1113 | 99/1 | 1.041 | 1.296 |
| HCFO-1131/HCFO-1131a | 99/1 | 1.042 | 1.294 |
| HCFO-1131/HFC-152a | 99/1 | 1.041 | 1.296 |
| HCFO-1131/HCFC-142b | 99/1 | 1.042 | 1.294 |
| HCFO-1131/HCFC-133a | 99/1 | 1.043 | 1.290 |
| HCFO-1131/HCFC-133 | 99/1 | 1.042 | 1.292 |
| HCFO-1131/HCFC-123 | 99/1 | 1.043 | 1.284 |
| HCFO-1131/HCFC-141b | 99/1 | 1.043 | 1.280 |
| HCFO-1131/HCFO-1130a | 99/1 | 1.043 | 1.277 |
| HCFO-1131/HCO-1130(E) | 99/1 | 1.044 | 1.273 |
| HCFO-1131/HCFC-142 | 99/1 | 1.043 | 1.282 |
| HCFO-1131/HCFC-142a | 99/1 | 1.043 | 1.289 |
| HCFO-1131/HCFO-1121 | 99/1 | 1.043 | 1.289 |
| HCFO-1131/HCFO-1121a | 99/1 | 1.043 | 1.289 |
| HCFO-1131/HCFC-132b | 99/1 | 1.043 | 1.277 |
| HCFO-1131/HCFC-132a | 99/1 | 1.045 | 1.274 |
| HCFO-1131/HCFC-132 | 99/1 | 1.043 | 1.274 |
| HCFO-1131/HCO-1130(Z) | 99/1 | 1.044 | 1.271 |
| HCFO-1131/HCFC-122 | 99/1 | 1.044 | 1.276 |
| HCFO-1131/HCC-140a | 99/1 | 1.044 | 1.273 |
| HCFO-1131/HCC-150a | 99/1 | 1.044 | 1.272 |
| HCFO-1131/HCFC-131a | 99/1 | 1.044 | 1.274 |
| HCFO-1131/HCO-1120 | 99/1 | 1.045 | 1.273 |
| HCFO-1131/HCFC-131 | 99/1 | 1.051 | 1.272 |
| HCFO-1131/HCC-140 | 99/1 | 1.049 | 1.271 |
| HCFO-1131/HCFC-121 | 99/1 | 1.045 | 1.276 |
| HCFO-1131HCO-1110 | 99/1 | 1.048 | 1.273 |
| HCFO-1131/HCFC-130a | 99/1 | 1.049 | 1.272 |
| HCFO-1131/HCC-130 | 99/1 | 1.051 | 1.268 |
| HCFO-1131/HCC-120 | 99/1 | 1.049 | 1.272 |
| HCFO-1131/ Tetrachloromethane | 99/1 | 1.044 | 1.275 |
| HCFO-1131/HCFC-131a | 99/1 | 1.051 | 1.272 |
| HCFO-1131/1-Chloropropene | 99/1 | 1.043 | 1.275 |
| HCFO-1131/HCFC-141 | 99/1 | 1.045 | 1.272 |
| HCFO-1131/HCFC-151 | 99/1 | 1.044 | 1.270 |
| HCFO-1131/HCFC-151a | 99/1 | 1.043 | 1.284 |
| HCFO-1131/HCFC-122b | 99/1 | 1.044 | 1.276 |
| HCFO-1131/HFO-1140 | 99/1 | 1.042 | 1.298 |
| HCFO-1131/HFO-1141 | 99/1 | 1.025 | 1.306 |
| HCFO-1131/water | 99/1 | 1.047 | 1.199 |
| HCFO-1131/HCl | 99/1 | 0.984 | 1.266 |
| HCFO-1131/HF | 99/1 | 1.043 | 1.267 |
| HCFO-1131/CO₂ | 99/1 | 0.980 | 1.253 |

## Claims

1. A composition comprising a refrigerant,
the refrigerant comprising 1,1,2-trifluoroethane (HFC-143), wherein
(1) the refrigerant further comprises a first additional compound and/or
(2) the composition further comprises at least one compound selected from the group consisting of water, hydrogen chloride, and hydrogen fluoride, and
the first additional compound is at least one compound selected from the group consisting of methane, tetrafluoroethane (HFC-14), difluoromethane (HFC-32), fluoromethane (HFC-41), trans-1,2-difluoroethylene (HFO-1132(E)), cis-1,2-difluoroethylene (HFO-1132(Z)), chloromethane (CH₃Cl), chlorotrifluoroethylene (HCFO-1113), 1,1-dichloro-1-fluoroethane (HCFC-141b), 1,1-dichloroethylene (HCO-1130a), trans-1,2-dichloroethylene (HCO-1130(E)), 1,2-dichloro-1-fluoroethylene (HCFO-1121), 1,1-dichloro-2-fluoroethylene (HCFO-1121a), 1,2-dichloro-1,1-difluoroethane (HCFC-132b), 1,1-dichloro-2,2-difluoroethane (HCFC-132a), 1,2-dichloro-1,2-difluoroethane (HCFC-132), cis-1,2-dichloroethylene (HCO-1130(Z)), 1,2,2-trichloro-1,1-difluoroethane (HCFC-122), 1,1,1-trichloroethane (HCC-140a), 1,1-dichloroethane (HCC-150a), 1,1,2-trichloro-1-fluoroethane (HCFC-131a), trichloroethylene (HCO-1120), 1,1,2-trichloro-2-fluoroethane (HCFC-131), 1,1,2-trichloroethane (HCC-140), 1,1,2,2-tetrachloro-1-fluoroethane (HCFC-121), tetrachloroethylene (HCO-1110), 1,1,1,2-tetrachloroethane (HCC-130a), 1,1,2,2-tetrachloroethane (HCC-130), 1,1,1,2,2-pentachloroethane (HCC-120), tetrachloromethane, 1,2,2-trichloro-1-fluoroethylene (HCFO-131a), 1-chloropropene, 2-chloropropene, 3-chloropropene, 1-chloro-2-fluoroethane (HCFC-151), 1-chloro-1-fluoroethane (HCFC-151a), 1,1,1-trichloro-2,2-difluoroethane (HCFC-122b), 1-chloroethylene (HCO-1140), and carbon dioxide.

2. A composition according to claim 1, wherein the refrigerant comprises HFC-143 in an amount of 30 mass% or more based on the entire refrigerant.

3. The composition according to claim 1, wherein the refrigerant further comprises a second additional compound, and the second additional compound is at least one compound selected from the group consisting of 1,1,2,2-tetrafluoromethane (HFC-134), 1-chloro-1,1-difluoroethane (HCFC-142b), and 1-chloro-2,2,2-trifluoroethane (HCFC-133a).

4. A composition comprising a refrigerant,
wherein
the refrigerant comprises 1-chloro-2-fluoroethylene (HCFO-1131),
(1) the refrigerant further comprises an additional compound and/or
(2) the composition further comprises at least one compound selected from the group consisting of water, hydrogen chloride, and hydrogen fluoride,
the additional compound is at least one compound selected from the group consisting of methane, HFC-14, trifluoromethane (HFC-23), 1,1-difluoroethylene (HFO-1132a), HFO-1132(E), 1,1,1-trifluoroethane (HFC-143a), HFO-1132(Z), 1,1,1,2-tetrafluoroethane (HFC-134a), chloromethane (CH₃Cl), 1,1,2,2-tetrafluoroethane (HFC-134), HCFC-1113, 1-chloro-1-fluoroethylene (HFO-1131a), 1,1-difluoroethane (HFC-152a), 1-chloro-1,1-fluoroethane (HCFC-142b), 1,1,2-trifluoroethane (HFC-143), 1-chloro-2,2,2-trifluoroethane (HFC-133a), 1-chloro-1,2,2-trifluoroethane (HFC-133), 1,1-chloro-2,2,2-trifluoroethane (HCFC-123), HCFC-141b, HCO-1130a, HCO-1130(E), 1-chloro-2,2-difluoroethane (HCFC-142), 1-chloro-1,2-difluoroethane (HCFC-142a), HCFO-1121, HCFO-1121a, HCFC-132b, HCFC-132a, HCFC-132, HCO-1130(Z), HCFC-122, HCC-140a, HCC-150a, HCFC-131a, HCO-1120, HCFC-131, HCC-140, HCFC-121, HCO-1110, HCC-130a, HCC-130, HCC-120 tetrachloromethane, HCFO-131a, 1-chloropropene, 2-chloropropene, 3-chloropropene, 1,2-dichloro-1-fluoroethylene (HCFO-141), HCFC-151, HCFC-151a, HCFC-122b, HCO-1140, 1-fluoroethylene (HFO-1141), and carbon dioxide, and
HFO-1131 is present in an amount of 30 mass% or more based on the entire refrigerant.

5. The composition according to any one of claims 1 to 4, comprising at least one compound selected from the group consisting of water, hydrogen chloride, and hydrogen fluoride.

6. Use of the composition according to any one of claims 1 to 5 as a refrigerant.

7. A refrigerating machine comprising the composition according to any one of claims 1 to 5 as a working fluid.

8. A method for operating a refrigerating machine, comprising the step of circulating the composition according to any one of claims 1 to 5 as a working fluid in a refrigerating machine.
